# EUROPEAN PATENT SPECIFICATION

(11) **EP 3 408 410 B1**
(45) Date of publication and mention of the grant of the patent: **20.12.2023**
(21) Application number: 17704609.1
(22) Date of filing: 25.01.2017
(51) Int. Cl.: C12Q 1/6806, C12Q 1/6841, G01N 1/31, G01N 35/00, G01N 1/28

(54) **PREDICTIVE DIAGNOSTIC WORKFLOW FOR TUMORS USING AUTOMATED DISSECTION, NEXT GENERATION SEQUENCING, AND AUTOMATED SLIDE STAINERS**
PRÄDIKTIVER DIAGNOSTISCHER ARBEITSFLUSS FÜR TUMORE MITHILFE AUTOMATISIERTER DISSEKTION, SEQUENZIERUNG DER NÄCHSTEN GENERATION UND AUTOMATISIERTE OBJEKTTRÄGERFÄRBER
FLUX DE TRAVAIL DE DIAGNOSTIC PRÉDICTIF POUR LES TUMEURS FAISANT APPEL À UNE DISSECTION AUTOMATISÉE, À UN SÉQUENÇAGE DE NOUVELLE GÉNÉRATION ET À DES DISPOSITIFS AUTOMATISÉS DE COLORATION DE LAME

(30) Priority: 26.01.2016 US 201662287182 P
(43) Date of publication of application: 05.12.2018
(73) Proprietor: Ventana Medical Systems, Inc., Tucson, Arizona 85755 (US)
(72) Inventor: GUSTAFSON, Heather, Tucson,AZ 85704 (US); HNATYSZYN, Harry James, Tucson,AZ 85737 (US)
(74) Representative: Teschemacher, Andrea
(86) International application number: PCT/US2017/014969
(87) International publication number: WO 2017/132276

(56) References cited:
- US-A1- 2015 361 508
- NIR PELED ET AL: "Next-Generation Sequencing Identifies and Immunohistochemistry Confirms a Novel Crizotinib-Sensitive ALK Rearrangement in a Patient with Metastatic Non-Small-Cell Lung Cancer", JOURNAL OF THORACIC ONCOLOGY, vol. 7, no. 9, 1 September 2012 (2012-09-01), pages e14-e16, XP055063267, ISSN: 1556-0864, DOI: 10.1097/JTO.0b013e3182614ab5
- ANDREA MAFFICINI ET AL: "Reporting Tumor Molecular Heterogeneity in Histopathological Diagnosis", PLOS ONE, vol. 9, no. 8, 15 August 2014 (2014-08-15) , page 1, XP055357016, DOI: 10.1371/journal.pone.0104979
- Thermofisher: "Uncovering tumor heterogeneity in FFPE samples by laser capture microdissection and next-generation sequencing", , 31 December 2015 (2015-12-31), XP055356504, Retrieved from the Internet: URL:https://tools.thermofisher.com/content /sfs/brochures/PG1395-PJ6555-CO29666-LCM-A pp-note-Focus-on-LCM-for-tumor-heterogenei ty-Global-FHR.pdf [retrieved on 2017-03-20]
- Soma Datta ET AL: "Laser capture microdissection: Big data from small samples", Histology and histopathology, 1 November 2015 (2015-11-01), page 1255, XP055356531, Spain DOI: 10.14670/HH-11-622 Retrieved from the Internet: URL:https://www.ncbi.nlm.nih.gov/pmc/artic les/PMC4665617/pdf/nihms707886.pdf
- M.-T. LIN ET AL: "Clinical Validation of KRAS, BRAF, and EGFR Mutation Detection Using Next-Generation Sequencing", AMERICAN JOURNAL OF CLINICAL PATHOLOGY, vol. 141, no. 6, 16 May 2014 (2014-05-16), pages 856-866, XP055358532, US ISSN: 0002-9173, DOI: 10.1309/AJCPMWGWGO34EGOD

## Description

### BACKGROUND OF THE INVENTION

### Field of the Invention

The invention relates to use of automated slide stainers, automated dissection tools, and/or next generation sequencers to assay predictive biomarkers and/or select therapies for cancers.

### Description of Related Art

In the last 20 years, cancer research and treatment has undergone a sea change. Whereas the first sequencing of the human genome cost on the order of $500 million to $1 billion, next generation sequencing (NGS) strategies have shrunk that cost to less than $2000, thereby permitting large-scale and relatively inexpensive evaluation of cancer genetics. *See* Reuter *et al.* These advances have accelerated the rate at which new therapeutic and diagnostic targets are identified and developed: in 2014 alone, the FDA approved a total of 51 new molecular entities and new biological products, many of which are targeted therapies. *See* FDA White Paper.

While such developments have certainly improved the treatment landscape, cancer remains remarkably resilient. Owing to their natural heterogeneity, tumors still frequently become resistant to previously effective therapeutics. *See* Sun & Yu. Diagnostic workflows to effectively and efficiently address this problem have yet to be realized.

Some diagnostic workflows have been identified using laser capture microdissection (LCM) and NGS. *See* Amemiya *et al*.; AB Brochure; Zhang *et al.* These procedures typically involve separately collecting tumor cells and non-tumor cells from the tumor using a LCM system, extracting genomic DNA (gDNA) or RNA from the collected cells, and sequencing specific loci to identify cancer-related mutations in the tumor cells and the normal cells. The mutations in the tumor cells are compared to the mutations in the non-tumor cells to identify mutations over-represented in the tumor cells. While these methods may provide a high-level view of mutations represented in the tumor, they do not provide any information about the spatial relationship of the mutations or how the mutations are inter-related from a functional standpoint.

Peled et al., Journal of thoracic oncology (2012), vol. 7, no. 9, page el 4-el 6 discloses a patient case study, wherein a first tumor sample from the pleural space is analyzed using FISH analysis prior to therapy onset. Upon determination of treatment failure, a second sample from a tumor biopsy is taken, nucleic acids are isolated therefrom and subsequently analyzed using various NGS methods. Finally, the results of the NGS analyses are confirmed on a further tumor sample using immunohistochemical staining and an alternative therapeutic agent is supplied to the patient.

Mafficini et al., PLOS ONE (2014), vol. 9, no. 8, page 1-10 discloses the use of targeted multigene next-generation sequencing (TM-NGS) to identify sections of tumor heterogeneity, wherein the TM-NGS findings are confirmed using immunohistochemical staining on further tumor sample. Treatment decisions are based on the confirmed biomarker findings.

### BRIEF SUMMARY OF THE INVENTION

This disclosure relates generally to use of automated dissection tools and/or next generation sequencing (NGS) platforms in cancer diagnostics and selection of therapeutics.

Methods are provided in which regions of a tumor sample predicted not to respond to a first therapeutic agent are excised from the sample with an automated dissection tool, mutations correlated with predictive biomarkers are detected in the excised region using NGS, and additional samples of the tumor are stained for one or more predictive biomarker(s) identified by NGS.

Systems for performing the methods described herein are provided comprising various combinations of cellular samples, nucleic acid samples, automated dissection tools, NGS systems, automated slide stainers, image scanners and analysis systems, and laboratory information systems.

Sets of diagnostic samples for use in the methods and systems disclosed herein are provided comprising slides containing cellular samples of a tumor and nucleic acid samples obtained from specific regions of such slides.

Other inventions and embodiments of the foregoing are set forth herein.

### BRIEF DESCRIPTION OF THE DRAWINGS

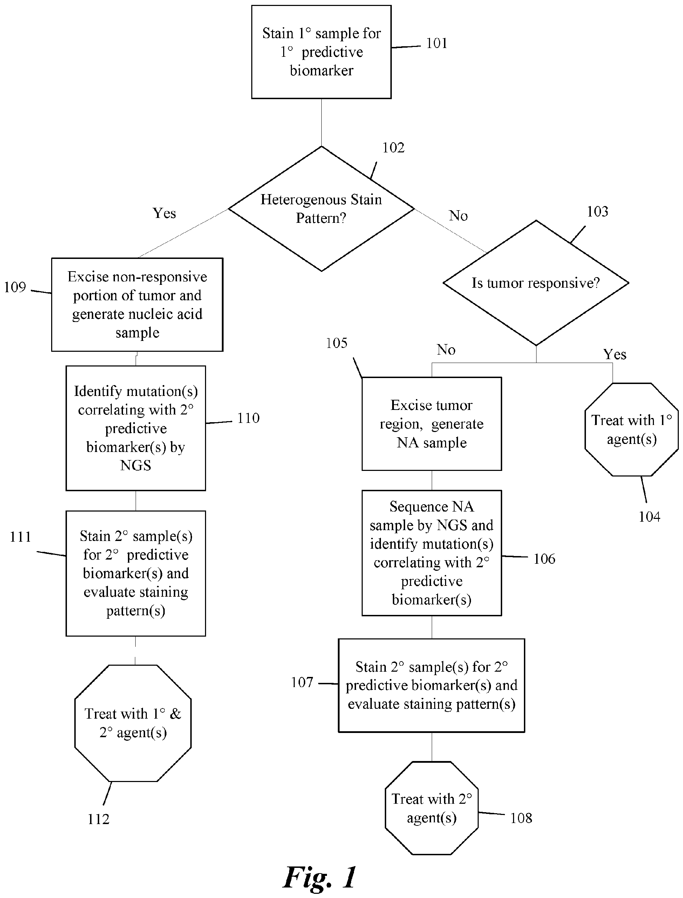

Fig. 1 is a flowchart demonstrating an exemplary workflow for the processes disclosed herein. Rectangular boxes indicate processes performed on samples. Diamond boxes indicate evaluation steps. Octagonal boxes indicate treatment decisions or treatment steps.

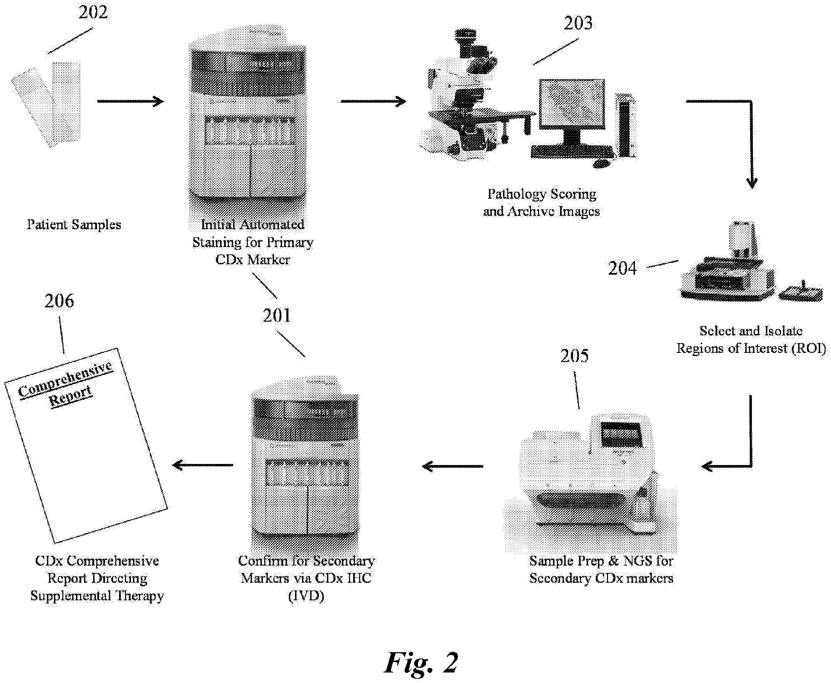

Fig. 2 is an exemplary system including an image analysis component as disclosed herein.

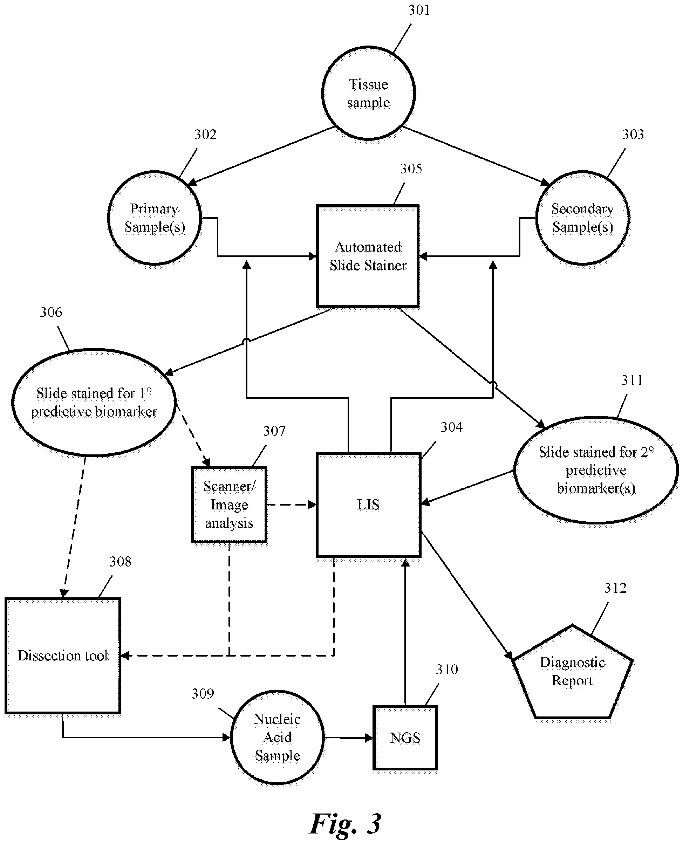

Fig. 3 is an exemplary system including a laboratory information system (LIS) and optional image analysis component as disclosed herein. Circles and ovals indicate potential sample components of the system. Squares and rectangles indicate sample manipulation, data analysis, and data storage components of the systems. Pentagonal shapes indicate data output from the system. Dashed lines indicate alternate workflows.

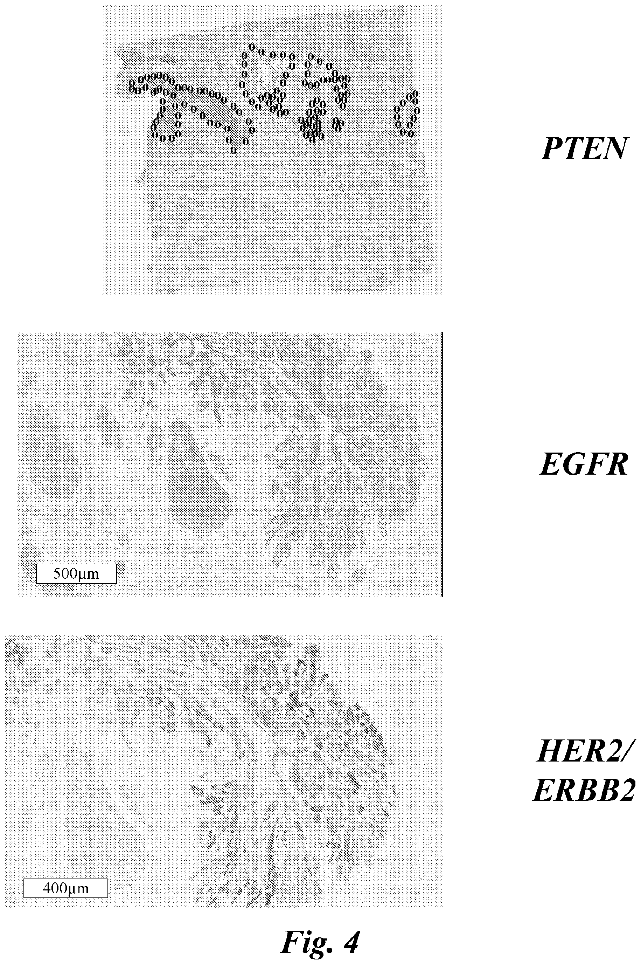

Fig. 4 is a series of IHC images from a prostate tumor evaluated according to a methods and system as disclosed herein. A primary sample was stained for PTEN. ROIs for excision and mutation analysis are outlined in the PTEN-stained image with the "o" symbol. Secondary samples were stained for EGFR and HER2.

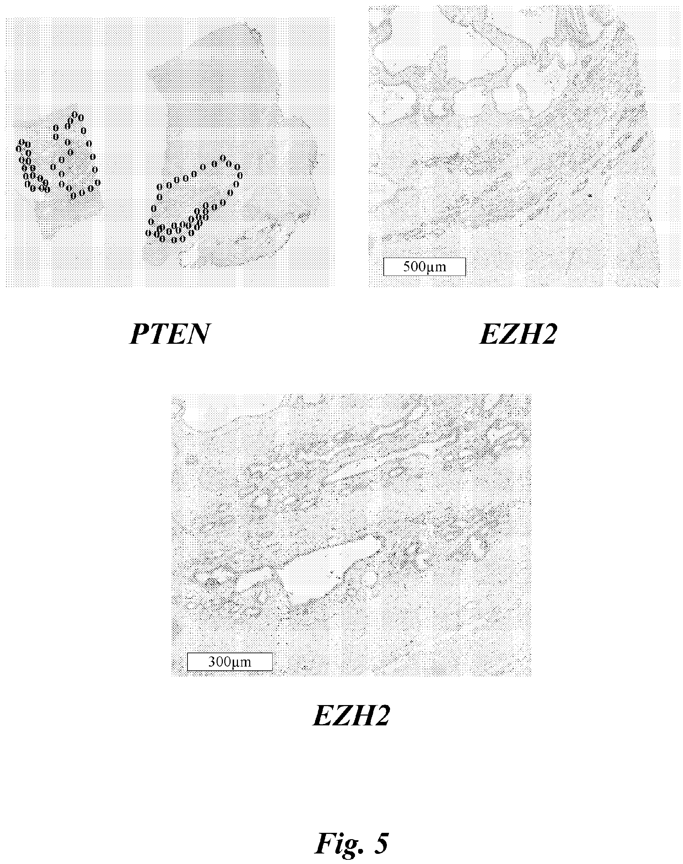

Fig. 5 is a series of IHC images from a prostate tumor evaluated according to a methods and system as disclosed herein. A primary sample was stained for PTEN. ROIs for excision and mutation analysis are outlined in the PTEN-stained image with the "o" symbol. Secondary samples were stained for EZH2.

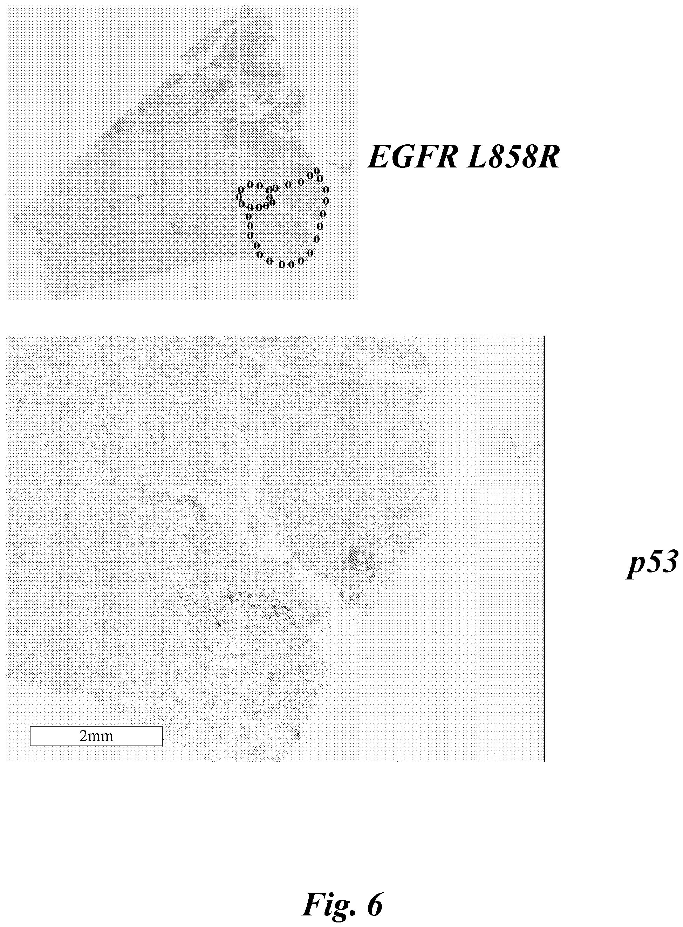

Fig. 6 is a series of IHC images from a lung tumor evaluated according to a methods and system as disclosed herein. A primary sample was stained for EGFR L858R. ROIs for excision and mutation analysis are outlined in the EGFR L858R-stained image with the "o" symbol. Secondary samples were stained for p53.

### DETAILED DESCRIPTION OF THE INVENTION

### I. Definitions

Unless defined otherwise, technical and scientific terms used herein have the same meaning as commonly understood by a person of ordinary skill in the art. See, *e.g*., Lackie, DICTIONARY OF CELL AND MOLECULAR BIOLOGY, Elsevier (4th ed. 2007); Sambrook et al., MOLECULAR CLONING, A LABORATORY MANUAL, Cold Springs Harbor Press (Cold Springs Harbor, N.Y. 1989). The term "a" or "an" is intended to mean "one or more." The terms "comprise," "comprises," and "comprising," when preceding the recitation of a step or an element, are intended to mean that the addition of further steps or elements is optional and not excluded.

**Antibody:** The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

**Antibody fragment:** An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.

**Biomarker:** As used herein, the term "biomarker" shall refer to any molecule or group of molecules found in a biological sample that can be used to characterize the biological sample or a subject from which the biological sample is obtained. For example, a biomarker may be a molecule or group of molecules whose presence, absence, or relative abundance is:
- characteristic of a particular cell or tissue type or state;
- characteristic of a particular pathological condition or state; or
- indicative of the severity of a disease state, the likelihood of progression or regression of the disease state, and/or the likelihood that the disease state will respond to a particular treatment.
As another example, the biomarker may be a cell type or a microorganism (such as a bacteria, mycobacteria, fungi, viruses, and the like), or a substituent molecule or group of molecules thereof.

**Biomarker-specific reagent:** A specific detection reagent that is capable of specifically binding directly to one or more biomarkers in the cellular sample, such as a primary antibody.

**Cellular sample:** As used herein, the term "cellular sample" refers to any sample containing intact cells, such as cell cultures, bodily fluid samples or surgical specimens taken for pathological, histological, or cytological interpretation.

**Detection reagent:** A "detection reagent" is any reagent that is used to deposit a stain in proximity to a biomarker-specific reagent in a cellular sample. Non-limiting examples include biomarker-specific reagents (such as primary antibodies), secondary detection reagents (such as secondary antibodies capable of binding to a primary antibody), tertiary detection reagents (such as tertiary antibodies capable of binding to secondary antibodies), enzymes directly or indirectly associated with the biomarker specific reagent, chemicals reactive with such enzymes to effect deposition of a fluorescent or chromogenic stain, and the like.

**Detectable moiety:** A molecule or material that can produce a detectable signal (such as visually, electronically or otherwise) that indicates the presence (i.e. qualitative analysis) and/or concentration (i.e. quantitative analysis) of the detectable moiety deposited on a sample. A detectable signal can be generated by any known or yet to be discovered mechanism including absorption, emission and/or scattering of a photon (including radio frequency, microwave frequency, infrared frequency, visible frequency and ultra-violet frequency photons). The term "detectable moiety" includes chromogenic, fluorescent, phosphorescent, and luminescent molecules and materials, catalysts (such as enzymes) that convert one substance into another substance to provide a detectable difference (such as by converting a colorless substance into a colored substance or vice versa, or by producing a precipitate or increasing sample turbidity). In some examples, the detectable moiety is a fluorophore, which belongs to several common chemical classes including coumarins, fluoresceins (or fluorescein derivatives and analogs), rhodamines, resorufins, luminophores and cyanines. Additional examples of fluorescent molecules can be found in Molecular Probes Handbook - A Guide to Fluorescent Probes and Labeling Technologies, Molecular Probes, Eugene, OR, TheroFisher Scientific, 11th Edition. In other embodiments, the detectable moiety is a molecule detectable via brightfield microscopy, such as dyes including diaminobenzidine (DAB), 4-(dimethylamino) azobenzene-4'-sulfonamide (DABSYL), tetramethylrhodamine (DISCOVERY Purple), N,N'-biscarboxypentyl-5,5'-disulfonato-indo-dicarbocyanine (Cy5), and Rhodamine 110 (Rhodamine).

**Histochemical detection:** A process involving staining a biomarker or other structures in a tissue sample with detection reagents in a manner that permits in a manner that permits microscopic detection of the biomarker or other structures in the context of the cross-sectional relationship between the structures of the tissue sample. Examples include immunohistochemistry (IHC), chromogenic *in situ* hybridization (CISH), fluorescent *in situ* hybridization (FISH), silver *in situ* hybridization (SISH), and hematoxylin and eosin (H&E) staining of formalin-fixed, paraffin-embedded tissue sections.

**Monoclonal antibody:** An antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, or a combination thereof.

**Predictive biomarker:** A biomarker whose staining pattern in a cellular sample is indicative of the likelihood that a particular treatment course will be effective or that other courses of treatment will not be effective.

**Sample:** As used herein, the term "sample" shall refer to any material obtained from a subject capable of being tested for the presence or absence of a biomarker.

**Secondary detection reagent:** A specific detection reagent capable of specifically binding to a biomarker-specific reagent.

**Section:** When used as a noun, a thin slice of a tissue sample suitable for microscopic analysis, typically cut using a microtome. When used as a verb, the process of generating a section.

**Serial section:** As used herein, the term "serial section" shall refer to any one of a series of sections cut in sequence by a microtome from a tissue sample. For two sections to be considered a "serial section" of one another, they do not necessarily need to be consecutive sections from the tissue, but they should generally contain the same tissue structures in the same cross-sectional relationship, such that the structures can be matched to one another via morphology.

**Specific detection reagent:** Any composition of matter that is capable of specifically binding to a target chemical structure in the context of a cellular sample. As used herein, the phrase "specific binding," "specifically binds to," or "specific for" or other similar iterations refers to measurable and reproducible interactions between a target and a specific detection reagent, which is determinative of the presence of the target in the presence of a heterogeneous population of molecules including biological molecules. For example, an antibody that specifically binds to a target is an antibody that binds this target with greater affinity, avidity, more readily, and/or with greater duration than it binds to other targets. In one embodiment, the extent of binding of a specific detection reagent to an unrelated target is less than about 10% of the binding of the antibody to the target as measured, e.g., by a radioimmunoassay (RIA). In certain embodiments, a biomarker-specific reagent that specifically binds to a target has a dissociation constant (Kd) of ≤1 µM, ≤100 nM, ≤10 nM, ≤1 nM, or ≤0.1 nM. In another embodiment, specific binding can include, but does not require exclusive binding. Exemplary specific detection reagents include nucleic acid probes specific for particular nucleotide sequences; antibodies and antigen binding fragments thereof; and engineered specific binding compositions, including ADNECTINs (scaffold based on 10th FN3 fibronectin; Bristol-Myers-Squibb Co.), AFFIBODYs (scaffold based on Z domain of protein A from S. aureus; Affibody AB, Solna, Sweden), AVIMERs (scaffold based on domain A/LDL receptor; Amgen, Thousand Oaks, CA), dAbs (scaffold based on VH or VL antibody domain; GlaxoSmithKline PLC, Cambridge, UK), DARPins (scaffold based on Ankyrin repeat proteins; Molecular Partners AG, Zürich, CH), ANTICALINs (scaffold based on lipocalins; Pieris AG, Freising, DE), NANOBODYs (scaffold based on VHH (camelid Ig); Ablynx N/V, Ghent, BE), TRANS-BODYs (scaffold based on Transferrin; Pfizer Inc., New York, NY), SMIPs (Emergent Biosolutions, Inc., Rockville, MD), and TETRANECTINs (scaffold based on C-type lectin domain (CTLD), tetranectin; Borean Pharma A/S, Aarhus, DK). Descriptions of such engineered specific binding structures are reviewed by Wurch et al., Development of Novel Protein Scaffolds as Alternatives to Whole Antibodies for Imaging and Therapy: Status on Discovery Research and Clinical Validation, Current Pharmaceutical Biotechnology, Vol. 9, pp. 502-509 (2008).

**Stain:** When used as a noun, the term "stain" shall refer to any substance that can be used to visualize specific molecules or structures in a cellular sample for microscopic analysis, including brightfield microscopy, fluorescent microscopy, electron microscopy, and the like. When used as a verb, the term "stain" shall refer to any process that results in deposition of a stain on a cellular sample.

**Subject:** As used herein, the term "subject" or "individual" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

**Tissue sample:** As used herein, the term "tissue sample" shall refer to a cellular sample that preserves the cross-sectional spatial relationship between the cells as they existed within the subject from which the sample was obtained.

**Tumor sample:** A cellular sample obtained from a tumor.

### II. Abbreviations

- 1°:: Primary
- 2°:: Secondary
- **5-FU:**: 5-fluorouracil
- **CISH:**: Chromogenic *in situ* hybridization
- **FFPE:**: Formalin-fixed, paraffin-embedded
- **FISH:**: Fluorescent *in situ* hybridization
- gDNA:: Genomic DNA
- **H&E:**: hematoxylin and eosin
- **IHC:**: Immunohistochemistry
- **ISH:**: In situ hybridization
- **LCM:**: Laser capture microdissection
- **LIS:**: Laboratory information system
- **MPS:**: Massively parallel sequencing
- **NA:**: Nucleic acid
- **NGS:**: Next generation sequencing
- **PCR:**: Polymerase chain reaction
- **ROI:**: Region of interest
- **SISH:**: Silver *in situ* hybridization
- **SNP:**: Single nucleotide polymorphism

### III. Methods of selecting cancer treatments and treating cancers.

In an embodiment, a treatment selection process is provided comprising histochemical staining, automated dissection, and next generation sequencing steps. The typical workflow for selecting a therapeutic course is illustrated at Fig. 1.

### A. Samples and sample staining

A tumor sample is obtained and a first portion of the tumor sample (hereafter termed a "primary sample" or 1° sample") is stained for a first predictive biomarker (also referred to as a "primary predictive biomarker" and a "1° predictive biomarker") for a first therapeutic agent 101.

Typically, the tumor sample is a tissue sample. In a specific embodiment, the tumor sample is a formalin-fixed, paraffin-embedded (FFPE) tissue samples. Any predictive biomarker may be used, whether now known or known in the future. For example, the predictive biomarker may be predictive of a response to a chemotherapy, to a targeted therapy, to a radiation therapy, or to a combination thereof. Exemplary predictive biomarkers are disclosed in Table 1:

**Table 1**

| **Biomarker** | **Biomarker Description** | **Therapeutic Agent** |
|---|---|---|
| ALK & ALK fusions | In certain tumors, Anaplastic Lymphoma Kinase (ALK) can be aberrantly expressed, often in the form of fusion proteins resulting from translocation events involving the *ALK* gene. | ALK inhibitor (crizotinib, alectinib) |
| | | HSP90 inhibitors (luminespib) |
| | | EGFR inhibitor (osimertinib, erlotinib, gefitinib) |
| AREG | Amphiregulin (AREG) is a secrete peptide hormone that acts as an activating ligand of EGFR. | anti-EGFR antibodies |
| | | EGFR inhibitor (osimertinib, erlotinib, gefitinib) |
| ATM | ATM is serine/threonine kinase involved in double stranded DNA damage repair response. Mutations in ATM leading to reduced function or loss of function are associated with several cancers. | DNA-damaging agents (platinum-based drugs, nucleoside analogs) |
| | | PARP inhibitor (olaparib) |
| | | ATR inhibitors (AZD6738) |
| | | CHK1 inhibitors (MK-8776) |
| BCL2 | BCL2 is an anti-apoptotic protein. Aberrant expression of BCL-2 (often due to translocations involving the *BCL2* gene) are associated with many cancers. | Bcl-2 inhibitor (venetoclax) |
| BRAF | BRAF is a serine/threonine kinase that plays a role in EGFR-mediated signal transduction. Constitutively active mutants of BRAF (such as V600E) are found in ~15% of all known human cancers. | anti-EGFR antibodies (cetuximab, panitumumab) |
| | | MEK inhibitor (tramatenib), B-Raf inhibitor (dabrafenib, vemurafenib) |
| | | Dasatinib |
| BRCA1 | BRCA1 is involved in many cellular functions, including DNA repair and cell-cycle checkpoint control. Cancer-associated mutations often result in loss of function. | PARP inhibitor (olaparib, rucaparib) |
| | | Chemotherapy (anthracyclines, CMF, taxanes) |
| c-KIT | c-KIT is a receptor tyrosine kinase that is involved in cell survival, proliferation, and differentiation. Cancer-associated mutations are typically activating or gain-of-function mutations. | tyrosine kinase inhibitor (imatinib mesylate, sunitinib) |
| CAIX | Carbonic anhydrase-IX (CAIX) is a zinc metalloenzymes that catalyzes the reversible hydration of carbon dioxide. CAIX is upregulated in some cancers. | IL-2 |
| CCR4 | C-C chemokine receptor type 4 (CCR4) is a G protein-coupled receptor family that is critical to T_{reg} cell migration. | anti-CCR4 (mogamulizumab) |
| CD30 | CD30 is a cell surface receptor expressed by activated, but not resting, T-cells and B-cells. CD30 expression is associated with some lymphomas. | anti-CD30 antibody-drug conjugate (brentuximab vedotin) |
| Claudin18.2 | Isoform 2 of claudin 18 (Claudin 18.2) is claudin-family protein overexpressed in many gastric tumors | Anti-claudin-18.2 antibody (IMAB362) |
| 17p 13.1 | Chromosome 17, region 17p13.1, encompasses several tumor suppressor genes, including TP53. Deletions in this region are associated with several cancers. | Bcl-2 inhibitor (venetoclax) |
| DLL3 | Delta-like 3 (DLL3) is a Notch-ligand that is predominantly expressed in fetal brain. Aberrant DLL3 expression is associated with some neuroendocrine tumors. | Anti-DLL3 antibody-drug conjugate (rovalpituzumab tesirine) |
| EGFR1 | Epidermal Growth Factor Receptor 1 (EGFR1) is an EGFR-family receptor involved in development and regulation of cellular proliferation, survival, and migration. EGFR1 amplification and over-expression is often observed in many cancers. | anti-EGFR antibodies (cetuximab, panitumumab) |
| | | EGFR inhibitor (osimertinib, erlotinib, gefitinib) |
| | | HER2/EGFR tki (afatinib) |
| Estrogen Receptor | Estrogen receptor (ER) is a nuclear receptor for estrogen. Two ER proteins are expressed by humans: ERα (encoded by *ESR1* gene) and ERβ (encoded by *ESR2* gene). ER is a dimer in activated form (which may be an αα, ββ, or αβ dimer). ER over-expression is associated with many cancers, including breast cancer, ovarian cancer, colon cancer, prostate cancer, and endometrial cancer | aromatase inhibitors (anastrozole, exemestane, letrozole) |
| | | selective ER modulators (tamoxifen, raloxifene, bazedoxifene) |
| | | selective estrogen receptor degrader (fulvestrant) |
| EREG | Epiregulin (EREG) is a secrete peptide hormone that acts as an activating ligand of EGFR. | anti-EGFR antibodies EGFR inhibitor (osimertinib, erlotinib, gefitinib) |
| ERCC1 | The *ERCC1* gene encodes DNA excision repair protein ERCC-1, which is involved in DNA repair and recombination. ERCC1 is often under-expressed or not expressed in cancer, frequently due to epigenetic changes, such as promoter methylation. | platinum-based chemotherapy (cisplatin) |
| FGF19 | Fibroblast growth factor 19 (FGF19) is a protein hormone that functions as a heparin-dependent ligand of FGF4. FGF19 is over-expressed in many cancers, including primary human hepatocellular carcinomas, lung squamous cell carcinomas, and colon adenocarcinomas. | FGFR4 inhibitor (BLU-554) |
| FGFR2 | Fibroblast growth factor receptor 2 is encoded by the *FGFR2* gene, consisting of 21 exons and encoding multiple splice variants. FGFR2b and FGFR2c isoforms are representative, each having extracellular three Ig-like domains, transmembrane domain, and cytoplasmic tyrosine kinase domain. FGFR2b differs from FGFR2c only in the latter half of the third Ig-like domain. FGFR2s function as a transmembrane receptor for FGF-family proteins. Missense mutations, amplifications, and intronic SNPs are associated with many cancer types. As used herein, "FGFR2" refers to the *FGFR2* gene, and any gene products thereof. | anti-FGFR2 (FPA144) |
| | | FGFR inhibitor (ARQ 087, Lucitanib, AZD4547, BGJ398, LY2874455, JNJ-42756493) |
| FGFR3 | Fibroblast growth factor receptor 3 is encoded by the *FGFR3* gene. FGFR3 is over-exprssed in many cancers | FGFR3 inhibitor (dovitinib, AZD4547) |
| | | TORC 1/2 inhibitor (CC-223) |
| FOLR1 | Folate receptor alpha is encoded by the *FOLR1* gene, and has a high affinity for folate. FOLR1 gene products are over-expressed in many epithelial-derived tumors. | FOLR1 Ab-drug conjugate (IMGN853) |
| HER2 | Human epidermal growth factor receptor 2 (HER2) is encoded by the *ERBB2* gene, and proto-oncogene. *ERBB2* is amplified or over-expressed in some cancers. | anti-HER2 (trastuzumab, pertuzumab, ado-trastuzumab emtansine) |
| KRAS | KRAS is a proto-oncogene GTPase encoded by the *KRAS* gene. Activating mutations have been identified in many cancers. | anti-EGFR (cetuzimab, panitumumab) |
| MGMT | O⁶ -Methylguanine-DNA-methyltransferase (MGMT) is a perotein encoded by the *MGMT* gene on chromosome 10. It is involved in a single-enzymatic DNA repair pathway. Loss of MGMT expression is associated with many cancers, including glioma, lymphoma, breast, and prostate cancer, and retinoblastoma. | 5-FU-based adjuvant therapy |
| MSLN | Mesothelin (MSLN) is encoded by the *MSLN* gene on chromosome 16. Its biological functionis unknown, but it isover-expressed in several tumors, including mesothelioma, and ovarian and pancreatic adenocarcinoma. | anti-MSLN antibody-drug conjugate (anetumab ravtansine) |
| p53 | p53 is a tumor suppressor encoded by the *TP53* gene on chromosome 17. Inactivating mutations of p53 are associated with severl cancers. | cisplatin-based chemotherapy |
| | | MDM2 antagonist |
| | | neoadjuvant radiation |
| MDM2 | E3 ubiquitin-protein ligase Mdm2 (MDM2) is encoded by the *MDM2* geneon chromosome 12. Increased expression of MDM2is associated with many tumors. | MDM2 antagonist |
| Progesterone receptor | Progesterone receptor (PR) is a nuclear receptor for the steroid hormone progesterone, encoded by the *PGR* gene residing on chromosome 1 1q22. PR over-expression is associated with many cancers, including breast cancer, ovarian cancer, colon cancer, prostate cancer, and endometrial cancer | progesterone anatagonists (mifepristone) |
| PD-L1 | Programmed death-ligand 1 (PD-L1), which is encoded by the *CD274* gene, induces suppression of T-cells via interaction with the PD-1 protein. Over-expression or aberrant expression of PD-L1 is frequently observed in numerous cancers and plays a role in immune avoidance by the tumor. | anti-PD-L1 (atezolizumab, durvalumab) |
| | | anti-PD-1 (nivolumab, pembrolizumab) |
| PDGFRB | Beta-type platelet-derived growth factor receptor (PDGFRB) is a protein that in humans is encoded by the *PDGFRB* gene. PDGFRB is over-expressed in many cancers. | tyrosine kinase inhibitor (imatinib mesylate) |
| PTEN | Phosphatase and tensin homolog (PTEN) is a protein encoded by the *PTEN* gene. Inactivating mutations are associated with development of many cancers. | anti-HER2 (trastuzumab, pertuzumab, ado-trastuzumab emtansine) |
| TP | Thymidine phosphorylase (TP) is a pentosyltransferases that plays a key role in pyrimidine salvage to recover nucleosides after DNA/RNA degradation and is also involved in angiogenesis. TP is unregulated in many cancers. | 5-FU- and capcetabine-based chemotherapy |

Many resources are available for identifying predictive biomarkers and their associated therapeutics. One example is the website "mycancergenome.org," which is maintained by the Vanderbilt-Ingram Cancer Center. Additionally, the FDA maintains a website with updated approvals of companion and complementary diagnostics. In a specific embodiment, at least the first predictive biomarker is predictive for a targeted therapy. In another embodiment, the predictive biomarker is a companion diagnostic for a targeted therapeutic.

The tumor samples are typically divided into several portions and affixed to a medium for microscopic analysis, such as a microscope slide. Where the sample is a tissue sample, the several portions may be tissue sections. In some embodiments, serial sections are taken from FFPE tissue samples. In some embodiments, serial sections are taken from a plurality of different sites of a FFPE block, which can be done to capture both intra-section heterogeneity and intra-block heterogeneity. In some embodiments, serial sections are taken from a plurality of different biopsy samples taken from different locations in the same tumor, which can be done to capture both intra-section heterogeneity and intra-tumor heterogeneity.

Staining is typically histochemical staining. Histochemical staining techniques typically involve contacting the sample with a biomarker-specific reagent under conditions sufficient to permit specific binding between the biomarker-specific reagent and the biomarker of interest. Binding of the biomarker-specific reagent to the biomarker facilitates deposition of a detectable moiety on the sample in proximity to locations containing the biomarker. The detectable moiety can be used to locate and/or quantify the biomarker to which the specific detection reagent is directed. Thereby, the presence and/or concentration of the target in a sample can be detected by detecting the signal produced by the detectable moiety.

In some embodiments, the detectable moiety is directly conjugated to the biomarker-specific reagent, and thus is deposited on the sample upon binding of the biomarker-specific reagent to its target (generally referred to as a direct labeling method). Direct labeling methods are often more directly quantifiable, but often suffer from a lack of sensitivity. In other embodiments, deposition of the detectable moiety is effected by the use of a detection reagent associated with the biomarker-specific reagent (generally referred to as an indirect labeling method). Indirect labeling methods have the increase the number of detectable moieties that can be deposited in proximity to the biomarker-specific reagent, and thus are often more sensitive than direct labeling methods, particularly when used in combination with dyes.

In some embodiments, an indirect method is used, wherein the detectable moiety is deposited via an enzymatic reaction localized to the biomarker-specific reagent. Suitable enzymes for such reactions are well-known and include, but are not limited to, oxidoreductases, hydrolases, and peroxidases. Specific enzymes explicitly included are horseradish peroxidase (HRP), alkaline phosphatase (AP), acid phosphatase, glucose oxidase, β-galactosidase, β-glucuronidase, and β-lactamase. The enzyme may be directly conjugated to the biomarker-specific reagent, or may be indirectly associated with the biomarker-specific reagent via a labeling conjugate. As used herein, a "labeling conjugate" comprises:
(a) a specific detection reagent; and
(b) an enzyme conjugated to the specific detection reagent, wherein the enzyme is reactive with the chromogenic substrate, signaling conjugate, or enzyme-reactive dye under appropriate reaction conditions to effect *in situ* generation of the dye and/or deposition of the dye on the tissue sample.

In non-limiting examples, the specific detection reagent of the labeling conjugate may be a secondary detection reagent (such as a species-specific secondary antibody bound to a primary antibody, an anti-hapten antibody bound to a hapten-conjugated biomarker-specific reagent, or a biotin-binding protein bound to a biotinylated biomarker-specific reagent), a tertiary detection reagent (such as a species-specific tertiary antibody bound to a secondary antibody, an anti-hapten antibody bound to a hapten-conjugated secondary biomarker-specific reagent, or a biotin-binding protein bound to a biotinylated secondary biomarker-specific reagent),or other such arrangements. An enzyme thus localized to the sample-bound biomarker-specific reagent can then be used in a number of schemes to deposit a detectable moiety.

In some cases, the enzyme reacts with a chromogenic compound/substrate. Particular non-limiting examples of chromogenic compounds/substrates include 4-nitrophenylphospate (pNPP), fast red, bromochloroindolyl phosphate (BCIP), nitro blue tetrazolium (NBT), BCIP/NBT, fast red, AP Orange, AP blue, tetramethylbenzidine (TMB), 2,2'-azino-di-[3-ethylbenzothiazoline sulphonate] (ABTS), o -dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-β-D-galactopyranoside (MU-Gal), p-nitrophenyl-α-D-galactopyranoside (PNP), 5-bromo-4-chloro-3-indolyl- β -D-glucuronide (X-Gluc), 3-amino-9-ethyl carbazol (AEC), fuchsin, iodonitrotetrazolium (INT), tetrazolium blue, or tetrazolium violet.

In some embodiments, the enzyme can be used in a metallographic detection scheme. Metallographic detection methods include using an enzyme such as alkaline phosphatase in combination with a water-soluble metal ion and a redox-inactive substrate of the enzyme. In some embodiments, the substrate is converted to a redox-active agent by the enzyme, and the redox-active agent reduces the metal ion, causing it to form a detectable precipitate. (see, for example, U.S. Patent Application No. 11/015,646, filed December 20, 2004, PCT Publication No. 2005/003777 and U.S. Patent Application Publication No. 2004/0265922).

Metallographic detection methods include using an oxido-reductase enzyme (such as horseradish peroxidase) along with a water soluble metal ion, an oxidizing agent and a reducing agent, again to for form a detectable precipitate. (See, for example, U.S. Patent No. 6,670,113).

In some embodiments, the enzymatic action occurs between the enzyme and the dye itself, wherein the reaction converts the dye from a non-binding species to a species deposited on the sample. For example, reaction of DAB with a peroxidase (such as horseradish peroxidase) oxidizes the DAB, causing it to precipitate.

In yet other embodiments, the detectable moiety is deposited via a signaling conjugate comprising a latent reactive moiety configured to react with the enzyme to form a reactive species that can bind to the sample or to other detection components. These reactive species are capable of reacting with the sample proximal to their generation, i.e. near the enzyme, but rapidly convert to a non-reactive species so that the signaling conjugate is not deposited at sites distal from the site at which the enzyme is deposited. Examples of latent reactive moieties include: quinone methide (QM) analogs, such as those described at WO2015124703A1, and tyramide conjugates, such as those described at, WO2012003476A2. In some examples, the latent reactive moiety is directly conjugated to a dye, such as N,N'-biscarboxypentyl-5,5'-disulfonato-indo-dicarbocyanine (Cy5), 4-(dimethylamino) azobenzene-4'-sulfonamide (DABSYL), tetramethylrhodamine (DISCO Purple), and Rhodamine 110 (Rhodamine). In other examples, the latent reactive moiety is conjugated to one member of a specific binding pair, and the dye is linked to the other member of the specific binding pair. In other examples, the latent reactive moiety is linked to one member of a specific binding pair, and an enzyme is linked to the other member of the specific binding pair, wherein the enzyme is (a) reactive with a chromogenic substrate to effect generation of the dye, or (b) reactive with a dye to effect deposition of the dye (such as DAB). Examples of specific binding pairs include:
(1) a biotin or a biotin derivative (such as desthiobiotin) linked to the latent reactive moiety, and a biotin-binding entity (such as avidin, streptavidin, deglycosylated avidin (such as NEUTRAVIDIN), or a biotin binding protein having a nitrated tyrosine at its biotin binding site (such as CAPTAVIDIN)) linked to a dye or to an enzyme reactive with a chromogenic substrate or
   reactive with a dye (for example, a peroxidase linked to the biotin-binding protein when the dye is DAB); and
(2) a hapten linked to the latent reactive moiety, and an anti-hapten antibody linked to a dye or to an enzyme reactive with a chromogenic substrate or reactive with a dye (for example, a peroxidase linked to the biotin-binding protein when the dye is DAB).

In a specific embodiment, the primary predictive biomarker is a protein and the staining method comprises a histochemical protein staining procedure (such as immunohistochemistry or an analogous procedure using other entities specific for the protein biomarker). In some embodiments, the primary predictive is a nucleic acid, and the staining method comprises a histochemical staining procedure using a nucleic acid probe (such as *in situ* hybridization (ISH)). In other embodiments, other types of biomarkers may be detected using specific binding agents for those biomarkers. For example, hyaluronan (HA) is an anionic, nonsulfated glycosaminoglycan that commonly accumulates in certain tumor types. HA typically is detected using an affinity histochemistry technique, wherein the specific binding agent is a fusion protein between an HA binding protein (such as hyaluronan binding protein (uniprot no. Q07021) or TNF-stimulated gene 6 (uniprot no. P98066)) and an immunoglobulin Fc region or a member of a specific binding pair (such as biotin).

Non-limiting examples of commercially available detection reagents or kits comprising detection reagents suitable for use with present methods include: VENTANA ultra View detection systems (secondary antibodies conjugated to enzymes, including HRP and AP); VENTANA iVIEW detection systems (biotinylated anti-species secondary antibodies and streptavidin-conjugated enzymes); VENTANA OptiView detection systems (OptiView) (anti-species secondary antibody conjugated to a hapten and an anti-hapten tertiary antibody conjugated to an enzyme multimer); VENTANA Amplification kit (unconjugated secondary antibodies, which can be used with any of the foregoing VENTANA detection systems to amplify the number of enzymes deposited at the site of primary antibody binding); VENTANA OptiView Amplification system (Anti-species secondary antibody conjugated to a hapten, an anti-hapten tertiary antibody conjugated to an enzyme multimer, and a tyramide conjugated to the same hapten. In use, the secondary antibody is contacted with the sample to effect binding to the primary antibody. Then the sample is incubated with the anti-hapten antibody to effect association of the enzyme to the secondary antibody. The sample is then incubated with the tyramide to effect deposition of additional hapten molecules. The sample is then incubated again with the anti-hapten antibody to effect deposition of additional enzyme molecules. The sample is then incubated with the detectable moiety to effect dye deposition); VENTANA ultra View ISH detection systems (for use with haptenated nucleic acid probes and anti-hapten primary antibodies; kit includes secondary antibodies conjugated to enzymes, including HRP and AP) ; VENTANA ISH iVIEW detection systems (for use with haptenated nucleic acid probes and anti-hapten primary antibodies; biotinylated anti-species secondary antibodies and streptavidin-conjugated enzymes); VENTANA DISCOVERY, DISCOVERY OmniMap, DISCOVERY UltraMap anti-hapten antibody, secondary antibody, chromogen, fluorophore, and dye kits, each of which are available from Ventana Medical Systems, Inc. (Tucson, Arizona); PowerVision and PowerVision+ IHC Detection Systems (secondary antibodies directly polymerized with HRP or AP into compact polymers bearing a high ratio of enzymes to antibodies); and DAKO EnVision^{™}+ System (enzyme labeled polymer that is conjugated to secondary antibodies).

### B. Heterogeneity analysis and treatment selection

Turning back to Fig. 1, once the primary sample is stained for a first predictive biomarker 101, it is evaluated for heterogeneity 102. In this context, heterogeneity is evaluated on the basis of whether different portions of the stained sample have staining patterns that indicate a difference in response to the primary agent. Where multiple sections from different locations in the tumor or different locations in a FFPE tissue block are evaluated for the first predictive biomarker, the sample shall be considered "heterogenous" if any section demonstrates a heterogenous staining pattern. In this context the term "primary agent" or "1° agent" shall refer to a therapeutic course for which the first predictive biomarker is predictive.

If the sample is not heterogenous (that is, the entire first portion of the tumor has a staining pattern indicting the same response), then the user evaluates the staining pattern to determine whether the tumor is likely to respond to the primary agent 103. If the answer is yes, then the subject is treated with the primary agent 104. If the answer is no, then a tumor area is marked as a region of interest (ROI) in the primary sample, the ROI is transferred to an automated dissection tool, the ROI is excised from a sufficient number of unstained serial sections of the primary sample with an automated dissection tool, a nucleic acid sample is generated from the excised sample 105. In this context, "sufficient number" shall mean at least enough sections to provide sufficient material to perform a next generation sequencing (NGS) process. The nucleic acid sample is evaluated by NGS for the presence of mutations correlating with additional predictive biomarkers (termed "secondary predictive biomarkers" or "2° predictive biomarkers") 106. A user (such as a pathologist) selects secondary predictive biomarkers based on the mutation analysis, additional portions of the tumor (termed "secondary samples" or "2° samples) are then stained for the secondary predictive biomarkers, and the staining pattern(s) are analyzed to determine whether the tumor is likely to respond to the secondary agent(s) 107. In this context the term "secondary agent" or "2° agent" shall refer to a therapeutic course for which the secondary predictive biomarker is predictive. The secondary agent(s) to which the tumor is likely to respond are selected as treatment candidates 108. In an embodiment, the secondary samples are serial sections of the first portion of the samples.

If the sample is heterogenous (that is, the first portion of the tumor has a staining pattern in at least one region that indicates the different response from the rest of the tumor), then the regions of the primary sample having a staining pattern indicating a lack of response are marked as an ROI, the ROI is transferred to an automated dissection tool, the ROI is excised from a sufficient number of unstained serial sections of the primary sample with an automated dissection tool, and a nucleic acid sample is generated from the excised sample 109. The nucleic acid sample is evaluated by NGS for the presence of mutations correlating with additional predictive biomarkers (termed "secondary predictive biomarkers" or "2° predictive biomarkers") 110. A user (such as a pathologist) selects secondary predictive biomarkers based on the mutation analysis, additional portions of the tumor (termed "secondary samples" or "2° samples) are stained for the secondary predictive biomarkers, and the staining pattern(s) are analyzed to determine whether the tumor is likely to respond to the secondary agent(s) 111. The primary agent and any secondary agent(s) to which the tumor is likely to respond are selected as treatment candidates 112. In an embodiment, the secondary samples are serial sections of the first portion of the samples.

### IV. Systems

In an embodiment, systems are provided for performing the methods described herein. In an embodiment, a system is provided including on one or more of an automated dissection apparatus, an NGS platform, and/or an automated slide staining platform, the system being adapted to perform the methods as described herein. The systems may also include an image analysis system for assessing staining patterns of stained slides and for capturing and storing images thereof, and/or LIS for tracking samples and workflows, storing diagnostic information about the samples, and/or tracking or providing instructions for assays to be performed on samples.

### A. Automated dissection tools

Automated dissection tools are devices that automatically excise tissue from slides. Typical automated dissection tools have two main components: (1) a tissue removal component that interacts with the tissue on the slide in a manner that precisely excises ROIs without substantially removing non-interested areas of the tissue; and (2) a computer-implemented guidance system that allows the user to select regions for excision in an image of the slide and guides the tissue removal component. Automated dissection tools generally fall into two categories: laser microdissection and mesodissection.

Laser microdissection tools typically comprise a microscope and a laser beam (with wavelengths in the infrared and/or ultraviolet range). A review of various laser microdissection technologies can be found at Legres *et al..* The user selects cells for excision from the guidance system, the laser cuts the area surrounding the ROI, and the cells of the ROI are removed. In an embodiment, the automated dissection tool is a laser microdissection tool.

Mesodissection tools essentially are tissue mills. In the typical design, a slide is placed on a stage that controls X and Y axis. The tissue is forced against a rotating cutting bit to cut the desired sections from the slide, and the cut sections are removed from the slide. An example of a mesodissection tool is described by Adey *et al.* In the example described by Adey, a cutting bit is used that simultaneously dispenses a liquid on the slide and aspirates the liquid from the slide. As the tissue is cut, it is suspended in the liquid and aspirated along with the aspirated liquid. A software system is provided that allows the user to digitally annotate the tissue sections for excision. In an embodiment, the automated dissection tool is a mesodissection tool.

### B. Next generation sequencing systems

As used herein, a "next generation sequencing platform" is any nucleic acid sequencing platform based on massively parallel sequencing (MPS): sequencing millions to billions of short read fragments (from 10s to 100s of bases in length) simultaneously. MPS typically can achieve an output of at least 10 Mbp per 18 hour cycle. NGS platforms can be broadly separated into categories on the basis of (1) template preparation method; and (2) process for performing MPS. Table 2 includes some examples of template preparation methods:

**Table 2**

| **Template preparation methods** | **Commercial Sequencers** |
|---|---|
| Emulsion PCR | ROCHE 454; Life Technologies Ion Proton; Life Technologies Ion Torrent |
| Clonal Bridge Amplification | Illumina MiSeq; Illumina HiSeq; Illumina Genome Analyzer IIX |
| Rolling circle amplification | Complete Genomics |
| Single molecule | Helicos Biosciences Heliscope; Pacific Biosciences SMRT |

Table 3 includes some examples of strategies for MPS:

**Table 3**

| **MPS Strategy** | | **Commercial Sequencers** |
|---|---|---|
| **Pyrosequencing** | **Description** | Templates are amplified using sequential addition of a single dNTPs. Pyrophosphate produced from the reaction is enzymatically converted proportionally to a visible signal, which is detected and recorded as a flowgram |
| | **Sequencers** | ROCHE 454 |
| **Cyclic reversible termination** | **Description** | "Bases are read using a cyclic reversible termination strategy, which sequences the template strand one nucleotide at a time through progressive rounds of base incorporation, washing, imaging and cleavage. In this strategy, fluorescently-labeled 3'-O-azidomethyldNTPs are used to pause the polymerization reaction, enabling removal of unincorporated bases and fluorescent imaging to determine the added nucleotide. Following scanning of the flow cell with a coupled-charge device (CCD) camera, the fluorescent moiety and the 3' block are removed, and the process is repeated." Reuter *et al.* |
| | **Sequencers** | Illumina MiSeq; Illumina HiSeq; Illumina Genome Analyzer IIX; Helicos Biosciences Heliscope |
| **semiconductor sequencing technology** | **Description** | "[Sequencing occurs by] a sequencing-by-synthesis reaction.... pH changes induced by the release of hydrogen ions during DNA extension. These pH changes are detected by a sensor ... and converted into a voltage signal. The voltage signal is proportional to the number of bases incorporated, and the sequential addition of individual nucleotides during each sequencing cycle allows base discrimination." Reuter *et al.* |
| | **Sequencers** | Life Technologies Ion Proton; Life Technologies Ion Torrent |
| **Phospholinked Fluorescent Nucleotides** | **Description** | "DNA synthesis occurs in zeptoliter-sized chambers, called zero-mode waveguides (ZMW), in which a single polymerase is immobilized at the bottom of the chamber. The physics of these chambers reduces background noise such that phosphate-labeled versions of all 4 nucleotides can be present simultaneously. Thus, polymerization occurs continuously, and the DNA sequence can be read in real-time from the fluorescent signals recorded in a video." Reuter *et al.* |
| | Sequencers | Pacific Biosciences SMRT |

In an embodiment, the NGS method includes one or more technologies from Table 2 or Table 3.

### C. Automated slide stainers

In some embodiments, the system includes an automated slide staining platform. Automated slide stainers typically include at least: reservoirs of the various reagents used in the staining protocols, a reagent dispense unit in fluid communication with the reservoirs for dispensing reagent to onto a slide, a waste removal system for removing used reagents and other waste from the slide, and a control system that coordinates the actions of the reagent dispense unit and waste removal system. In addition to performing staining steps, many automated slide stainers can also perform steps ancillary to staining (or are compatible with separate systems that perform such ancillary steps), including: slide baking (for adhering the sample to the slide), dewaxing (also referred to as deparaffinization), antigen retrieval, counterstaining, dehydration and clearing, and coverslipping. The Prichard reference describes several specific examples of automated IHC/ISH slide stainers and their various features, including the intelliPATH (Biocare Medical), WAVE (Celerus Diagnostics), DAKO OMNIS and DAKO AUTOSTAINER LINK 48 (Agilent Technologies), BENCHMARK (Ventana Medical Systems, Inc.), Leica BOND, and Lab Vision Autostainer (Thermo Scientific) automated slide stainers. Additionally, Ventana Medical Systems, Inc. is the assignee of a number of United States patents disclosing systems and methods for performing automated analyses, including U.S. Pat. Nos. 5,650,327, 5,654,200, 6,296,809, 6,352,861, 6,827,901 and 6,943,029, and U.S. Published Patent Application Nos. 20030211630 and 20040052685.

Commercially-available staining units typically operate on one of the following principles: (1) open individual slide staining, in which slides are positioned horizontally and reagents are dispensed as a puddle on the surface of the slide containing a tissue sample (such as implemented on the DAKO AUTOSTAINER Link 48 (Agilent Technologies) and intelliPATH (Biocare Medical) stainers); (2) liquid overlay technology, in which reagents are either covered with or dispensed through an inert fluid layer deposited over the sample (such as implemented on VENTANA BenchMark and DISCOVERY stainers); (3) capillary gap staining, in which the slide surface is placed in proximity to another surface (which may be another slide or a coverplate) to create a narrow gap, through which capillary forces draw up and keep liquid reagents in contact with the samples (such as the staining principles used by DAKO TECHMATE, Leica BOND, and DAKO OMNIS stainers). Some iterations of capillary gap staining do not mix the fluids in the gap (such as on the DAKO TECHMATE and the Leica BOND). In variations of capillary gap staining termed dynamic gap staining, capillary forces are used to apply sample to the slide, and then the parallel surfaces are translated relative to one another to agitate the reagents during incubation to effect reagent mixing (such as the staining principles implemented on DAKO OMNIS slide stainers (Agilent)). In translating gap staining, a translatable head is positioned over the slide. A lower surface of the head is spaced apart from the slide by a first gap sufficiently small to allow a meniscus of liquid to form from liquid on the slide during translation of the slide. A mixing extension having a lateral dimension less than the width of a slide extends from the lower surface of the translatable head to define a second gap smaller than the first gap between the mixing extension and the slide. During translation of the head, the lateral dimension of the mixing extension is sufficient to generate lateral movement in the liquid on the slide in a direction generally extending from the second gap to the first gap. *See* WO 2011-139978 A1. It has recently been proposed to use inkjet technology to deposit reagents on slides. *See* WO 2016-170008 A1. This list of automated staining technologies is not intended to be comprehensive, and any fully or semi-automated system for performing biomarker staining may be used.

### D. Image analysis systems

In some embodiments, digital images of the stained slides are analyzed instead of (or in addition to) live reading on a microscope. In such embodiments, the stained slides can be imaged on a imager slide scanner. At a basic level, slide scanners generate a representative digital image of the stained sample. The typical slide scanner includes at least: (1) a microscope with lens objectives, (2) a light source (such as halogen, light emitting diode, white light, and/or multispectral light sources), (3) robotics to move glass slides around (or to move the optics around the slide), (4) one or more digital cameras for image capture, (5) a computer and associated software to control the robotics and to manipulate, manage, and view digital slides. Digital data at a number of different X-Y locations (and in some cases, at multiple Z planes) on the slide are captured by the camera's charge-coupled device (CCD), and the images are joined together to form a composite image of the entire scanned surface. Common methods to accomplish include:
(1) Tile based scanning, in which the slide stage or the optics are moved in very small increments to capture square image frames, which overlap adjacent squares to a slight degree. The captured squares are then automatically matched to one another to build the composite image; and
(2) Line-based scanning, in which the slide stage moves in a single axis during acquisition to capture a number of composite image "strips." The image strips can then be matched with one another to form the larger composite image. In some cases,

A detailed overview of various slide scanners can be found at Farahani *et al.* Examples of slide scanners include: 3DHistech PANNORAMIC SCAN II; DigiPath PATHSCOPE; Hamamatsu NANOZOOMER RS, HT, and XR; Huron TISSUESCOPE 4000, 4000XT, and HS; Leica SCANSCOPE AT, AT2, CS, FL, and SCN400; Mikroscan D2; Olympus VS120-SL; Omnyx VL4, and VL120; PerkinElmer LAMINA; Philips ULTRA-FAST SCANNER; Sakura Finetek VISIONTEK; Unic PRECICE 500, and PRECICE 600x; VENTANA ISCAN COREO and ISCAN HT; and Zeiss AXIO SCAN.Z1. Other exemplary systems and features can be found in, for example, International Patent Application No.: PCT/US2010/002772 (Patent Publication No.: WO/2011/049608) entitled IMAGING SYSTEM AND TECHNIQUES or disclosed in U.S. Patent Application No. 61/533,114, filed on Sep. 9, 2011, entitled IMAGING SYSTEMS, CASSETTES, AND METHODS OF USING THE SAME. International Patent Application No. PCT/US2010/002772 and U.S. Patent Application No. 61/533,114.

In some embodiments, the image analysis system is integrated with the automated dissection tool, such that ROIs identified by the pathologist in the image analysis system may be transferred directly to the automated dissection tool for identification of regions to be excised from the slide. In other embodiments, the image analysis system is adapted only for diagnostic evaluation of the microscope slides, and a separate imaging system is integrated with the automated dissection tool for identifying ROIs and directing the excision thereof.

An exemplary system including an image analysis system is illustrated at Fig. 2. An automated slide stainer 201 is provided to stain the primary slide of a set of slides from a single sample 202. The stained slide is scanned by the image analysis system 203, and the pathologist reviews the staining pattern and determines (a) whether the sample is heterogenous, and (b) whether any portion of the sample is likely to respond to the drug for which the primary biomarker is diagnostic. The pathologist manually marks any non-responsive regions as an ROI in the image analysis system 203, which is transferred to an automated dissection tool 204. Unstained slides from the set of slides containing serial sections of the primary slide are transferred to the automated dissection tool 204, the ROI is matched to the unstained sections, and the ROI is excised. The excised portion of the sample is processed to obtain a nucleic acid sample and the nucleic acid sample is sequenced on a NGS sequencer 205. A software suite is used to identify mutations associated with the sample, and the user selects one or more of the mutations that is associated with a predictive biomarker. Unstained slides from the set of slides 202 are passed to the automated slide stainer 201 (which may be the same or different from the automated slide stainer that stained the primary slide). The automated slide stainer 201 stains the unstained slides with biomarker-specific reagents for the predictive biomarkers selected by the user. If desired, the slides stained with the secondary predictive biomarkers may be evaluated on the image analysis system 203. When all slides have been evaluated, a diagnostic report 206 including the prediction for each predictive biomarker is generated, from which the treating physician may make diagnostic and therapeutic decisions.

### E. Laboratory information systems

The system may further include a LIS. LIS typically performs one or more functions selected from: recording and tracking processes performed on samples and on slides and images derived from the samples, instructing different components of the system to perform specific processes on the samples, slides, and/or images, and track information about specific reagents applied to samples and or slides (such as lot numbers, expiration dates, volumes dispensed, etc.). LIS usually comprises at least a database containing information about samples; labels associated with samples, slides, and/or image files (such as barcodes (including 1-dimensional barcodes and 2-dimensional barcodes), radio frequency identification (RFID) tags, alpha-numeric codes affixed to the sample, and the like); and a communication device that reads the label on the sample or slide and/or communicates information about the slide between the LIS and the other components of the immune context scoring system. Thus, for example, a communication device could be placed at each of a sample processing station, automated slide stainer, automated dissection tool, and NGS system. When the sample is initially processed into sections, information about the sample (such as patient ID, sample type, processes to be performed on the section(s)) may be entered into the communication device, and a label is created for each section generated from the sample. At each subsequent station, the label is entered into the communication device (such as by scanning a barcode or RFID tag or by manually entering the alpha-numeric code), and the station electronically communicates with the database to, for example, instruct the station or station operator to perform a specific process on the section and/or to record processes being performed on the section. At a scanning platform, the scanning platform may also encode each image with a computer-readable label or code that correlates back to the section or sample from which the image is derived, such that when the image is sent to the image analysis system, image processing steps to be performed may be sent from the database of LIS to the image analysis system and/or image processing steps performed on the image by image analysis system are recorded by database of LIS. Commercially available LIS systems useful in the present methods and systems include, for example, VENTANA Vantage Workflow system (Roche).

An exemplary system including an LIS and an optional image analysis system is illustrated at Fig. 3. A tissue sample 301 is divided into a plurality of samples, including at least one primary sample 302 mounted on a microscope slide and a set of secondary samples 303, also mounted on microscope slides. Instructions to stain the primary sample 302 for a first predictive biomarker are recorded into an LIS 304 and input into an automated slide stainer 305. In some embodiments, the instructions are sent from the LIS 304 to the automated slide stainer 305 and automatically implemented on the automated slide stainer 305. In some embodiments, the LIS 304 generates a label associated with the primary sample 302. In some embodiments, the label bears the instructions imprinted on the label, which the user may manually enter into the automated slide stainer 305. In other embodiments, the label includes a designation (such as a barcodes (including 1-dimensional barcodes and 2-dimensional barcodes), radio frequency identification (RFID) tags, alpha-numeric codes, and the like) readable by a station at the automated slides stainer to either to directly instruct the automated slide stainer 305 of the program to implement on the slide, or to inform the user of how to program the automated slide stainer 305. The slide is stained by the automated slide stainer 305 for the first predictive biomarker to obtain the primary stained slide 306. If evaluation on an image analysis system is desired, the primary stained slide 306 is scanned by the image analysis system 307, and the pathologist reviews the staining pattern and determines (a) whether the sample is heterogenous, and (b) whether any portion of the sample is likely to respond to the drug for which the primary biomarker is diagnostic. If image analysis is not desired, the pathologist performs the same analysis on a microscope. In either case, the pathologist's analysis is recorded in the LIS 304. Where image analysis is performed, digital images of the primary stained slide 306 may also be recorded in the LIS 304, which may also be manually annotated to identify the ROI. The primary stained slide 306 is then transferred to the automated dissection tool 308, and the ROI is identified in a sufficient number of unstained serial sections of the primary sample 302 and excised. In some embodiments, the ROI is identified *de novo* on the automated dissection tool 308, for example, by a technician or pathologist identifying morphological structures in the unstained section that correlate to the ROI from the primary slide. In other embodiments, the ROI is transferred to the automated dissection tool 308 from the image analysis system 307 or the LIS 304, which may then be modified or accepted by the user. The precise portion which is excised may be recorded in the LIS 304. The excised portion of the sample is processed to obtain a nucleic acid sample 309 and the nucleic acid sample is sequenced on a NGS sequencer 310. A software suite associated with the NGS identifies mutations associated with the sample, and the user selects one or more of the mutations that is associated with a predictive biomarker. The mutations identified by the NGS 310 and the secondary predictive biomarkers selected by the user may be stored in the LIS 304. Instructions to stain the secondary sample(s) 303 for additional predictive biomarker(s) are recorded into the LIS 304 and input into an automated slide stainer 305 (which may be the same or different from the automated slide stainer that stained the primary slide). In some embodiments, the instructions are sent from the LIS 304 to the automated slide stainer 305 and automatically implemented on the automated slide stainer 305. In some embodiments, the LIS 304 generates a label associated with the primary sample 302. In some embodiments, the label bears the instructions imprinted on the label, which the user may manually enter into the automated slide stainer 305. In other embodiments, the label includes a designation (such as a barcodes (including 1-dimensional barcodes and 2-dimensional barcodes), radio frequency identification (RFID) tags, alpha-numeric codes, and the like) readable by a station at the automated slides stainer to either to directly instruct the automated slide stainer 305 of the program to implement on the slide, or to inform the user of how to program the automated slide stainer 305. The slide is stained by the automated slide stainer 305 for the additional predictive biomarker(s) to obtain the secondary stained slides 311. If image analysis is desired, the secondary stained slide(s) 306 is/are scanned by the image analysis system 307, and the pathologist reviews the staining pattern and determines whether any portion of the sample is likely to respond to the drug(s) for which the secondary biomarker(s) is/are diagnostic. If image analysis is not desired, the pathologist performs the same analysis on a microscope. In either case, the pathologist's analysis is recorded in the LIS 304. Where image analysis is performed, digital images of the secondary stained slide(s) 311 may also be recorded in the LIS 304. When all slides have been analyzed, a diagnostic report 312 including the evaluation for each predictive biomarker is generated, from which the treating physician may make diagnostic and therapeutic decisions.

### V. Examples

To test feasibility of the described methods and workflows, and to determine if it could be used to gain predictive diagnostic information about a case, a model system was developed using PTEN or EGFR as a primary predictive biomarker. All histochemical stains were performed on a VENTANA BenchMark ULTRA IHC/ISH slide stainer. ROI identification and excision was performed using a ROCHE Automated Dissection Tool mesodissection instrument. DNA was isolated using Roche MagNA Pure96, and quality control of the thus obtained DNA sample was performed by qPCR utilizing a Roche Lightcycler 480. 10ng of DNA was obtained for highest library prep success rate (1.6ng/µL preferred). A total number of slides necessary for each ROI was calculated based on the assumption that ~250mm² of tissue would be required. Targeted sequencing was performed on a Life Technologies ION TORRENT PGM NGS platform utilizing Life Technology Cancer HotSpot Panel v2 and the on-system variant analysis.

In a first example, a prostate tumor sample was stained for PTEN via IHC. Positively-stained tumor regions were isolated and sequenced using only a filter for coverage and non-synonymous mutations. Results are shown at Table 4:

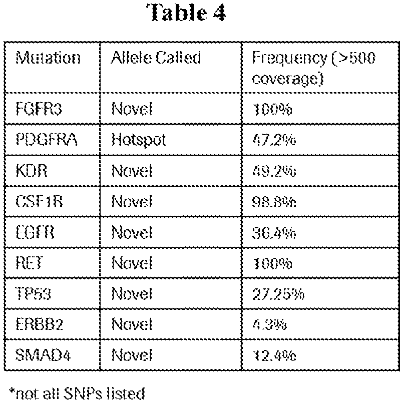

For quality purposes only mutations that had coverage great than 500X (meaning that SNP was sequenced a least 500 times) were recorded. From this list, IHC tests are commercially available for EGFR expression and HER2 which is encoded by the ERBB2 gene. Using two more slides for these tests, over-expression of EGFR and unexpected expression of HER2 in a prostate cancer case were observed, both of which could identify additional treatment targets.

A second prostate case was again stained for PTEN by IHC, the image of which can be seen at Fig. 5. A section of positively-staining sample was sequenced, the results of which are displayed at Table 5:

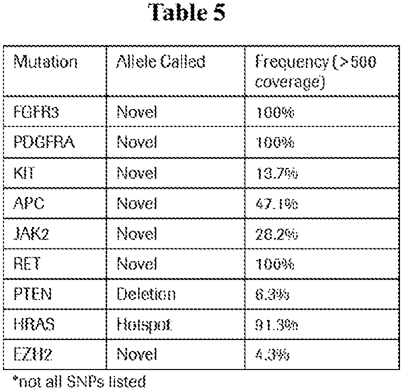

Additional sections were stained for EZH2, results of which can be seen at Fig. 5. c-Kit was additionally recognized as a potential predictive biomarker. However, IHC stain revealed no staining of interest. This illustrates the point that the presence of a mutation correlating with a predictive biomarker does not necessary provide a clinically actionable course of treatment.

A lung case was stained via IHC for a single nucleotide polymorphism of EGFR: EGFR L858R. Image is displayed at Fig. 6. A region negative for the mutation was excised and sequenced. Results are shown at Table 6. A p53 IHC stain was performed, an image of which can be seen at Fig. 6. The stained slide demonstrated a loss of p53 expression throughout, which is predictive for cisplatin-based chemotherapy and MDM2 antagonists.

The lung case was also used to test the necessity of the dissection tool as opposed to sequencing the whole sample. As can be seen at Table 7, some mutations that are detectable in excised portions of the sample can be lost if the whole sample is sequenced. This shows the importance of sequencing annotated tumor regions rather than the whole tissue.

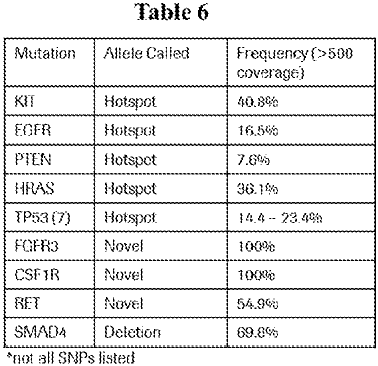

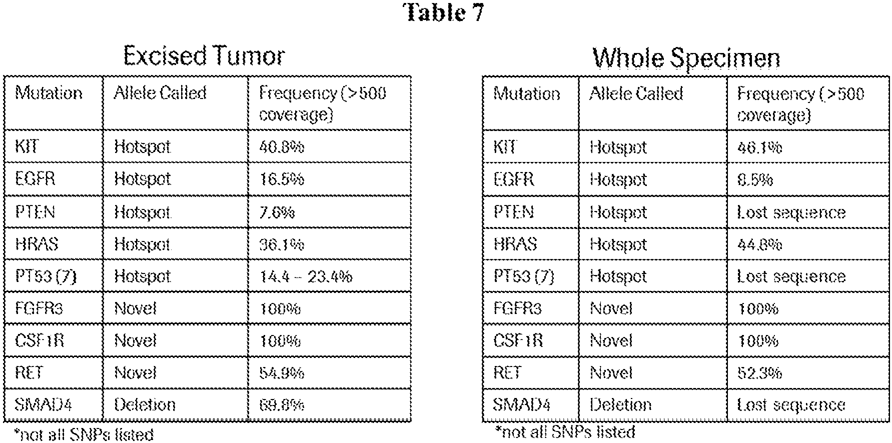

### VII. References

Adey et al., A mill based instrument and software system for dissecting slide-mounted tissue that provides digital guidance and documentation, BMC Clinical Pathology, Vol. 13, Issue 29 (2013).
Amemiya, An approach for analyzing genetic heterogeneity in retrospective tumor samples using laser capture microdissection, real-time PCR, and next-generation sequencing (2015), https://tools.thermofisher.com/content/sfs/brochures/PG1345-PJ5184-CO34268-VariantDetectionAnalysisLCM AmpliSeq_qPCR Americas FLR.pdf
Applied Biosystems, Inc., Uncovering tumor heterogeneity in FFPE samples by laser capture microdissection and next-generation sequencing (2015), https://tools.thermofisher.com/content/sfs/brochures/PG1395-PJ6555-CO29666-LCM-App-note-Focus-on-LCM-for-tumor-heterogeneity-Global-FHR.pdf ("AB Brochure").
Farahani et al., Whole slide imaging in pathology: advantages, limitations, and emerging perspectives, Pathology and Laboratory Medicine Int'1, Vol. 7, p. 23-33 (June 2015).
White Paper: FDA and Accelerating the Development of the New Pharmaceutical Therapies, available at http://www.fda.gov/downloads/AboutFDA/ReportsManualsForms/ Reports/UCM439183.pdf ("FDA White Paper").
Legeres et al., Beyond laser microdissection technology: follow the yellow brick road for cancer research, Am J Cancer Res. Vol. 4, Issue 1, pp. 1-28 (2014).
Prichard, Overview of Automated Immunohistochemistry, Arch Pathol Lab Med., Vol. 138, pp. 1578-1582 (2014).
Reuter et al., High-Throughput Sequencing Technologies, Molecular Cell, Vol. 58, issue 4, pp. 586-597, (May 21, 2015).
Sun & Yu, Intra-tumor heterogeneity of cancer cells and its implications for cancer treatment, Acta Pharmacol Sin. Vol. 36, Issue 10, pp 1219-1227 (Oct. 2015).
Zhang et al., Profiling Cancer Gene Mutations in Clinical Formalin-Fixed, Paraffin-Embedded Colorectal Tumor Specimens Using Targeted Next-Generation Sequencing, The Oncologist, Vol. 19, No. 4, pp 336-342 (2014).

## Claims

1. A method comprising:
histochemically staining a first sample of a tumor for a first predictive biomarker for a first therapeutic agent;
excising one or more region(s) from the first sample with an automated dissection tool, wherein the excised region has a staining pattern for the first predictive biomarker indicating that the region is unlikely to respond to the first therapeutic agent;
detecting with a next generation sequencer one or more mutations predictive of a response to one or more additional therapeutic agents in a nucleic acid sample derived from the excised region(s) of the first sample;
staining one or more additional samples of the tumor for one or more additional predictive biomarker(s) correlating to the one or more mutations identified in the samples, the one or more additional predictive biomarkers being predictive of a response to one or more of the additional therapeutic agent(s).

2. The method of claim 1, further comprising:
generating a report identifying a therapeutic course for the subject, said therapeutic course comprising administering to the subject:
the first therapeutic agent if at least one region of the first sample has a staining pattern for the first predictive biomarker indicating that at least a portion of the tumor is likely to respond to the first therapeutic agent; and
one or more of the additional therapeutic agent(s) if at least one region of the additional sample(s) has a staining pattern for the corresponding additional predictive biomarker indicating that at least a portion of the tumor is likely to respond to the additional therapeutic agent.

3. The method of claim 1 or 2, wherein the tumor is a solid tumor.

4. The method of claim 3, wherein the solid tumor is a formalin-fixed, paraffin-embedded (FFPE) tissue sample, and the first sample and the additional sample(s) are microtome sections of the FFPE tissue sample.

5. The method of claim 4, wherein first sample and the additional sample(s) are serial sections.

6. A system comprising:
(a) a set of microscope slides comprising:
(a1) a first microscope slide having deposited thereon a first sample of a tumor, wherein the first sample is histochemically stained for a first predictive biomarker for a first therapeutic agent;
(a2) one or more additional unstained microscope slides having deposited thereon an additional sample of the tumor;
(b) an image analysis system for identifying one or more regions of the first sample having a staining pattern for the first predictive biomarker indicating that at least a portion of the tumor is unlikely to respond to the first therapeutic agent;
(c) an automated dissection tool programmed to excise the one or more regions of the first sample having a staining pattern for the first predictive biomarker characteristic of a lack of response to the first therapeutic agent from the first sample;
(d) a next generation sequencer programmed to identify the presence or absence of mutations correlated with one or more additional predictive biomarkers in a nucleic acid sample derived from the regions of the first sample excised by the automated dissection tool; and
(e) an automated slide stainer programmed to stain the additional slide(s) with one or more of the additional predictive biomarker(s).

7. The system of claim 6, further comprising:
(f) a laboratory information system (LIS) comprising a database, the database containing:
(f1) a mutation analysis of the nucleic acid sample by the next generation sequencer, wherein the mutation analysis indicates at least the presence or absence of mutations in the nucleic acid sample correlating to one or more additional predictive biomarker(s) for one or more additional therapeutic agent(s); and
(f2) instructions for directing the automated slide stainer to stain the second sample of the tumor with the one or more additional predictive biomarkers identified by the mutation analysis.

8. The system of claim 7, wherein at least one of the unstained microscope slides has affixed thereto a label generated by the LIS and readable by the automated slide stainer, wherein the label identifies the slide as being appropriate for execution of the instructions of (f2) by the automated slide stainer.

9. The system of claim 8, wherein the label automatically directs the automated slide stainer to execute the instructions on the second sample.

10. The system of claim 8, wherein the label generates a report for an operator of the automated slide stainer, the report instructing the manual operator to program the automated slide stainer to execute the instructions on the second sample.

11. A set of diagnostic samples derived from a tumor, said set of diagnostic samples comprising:
(a) a first sample of a tumor, wherein the first sample is stained for a first predictive biomarker for a first therapeutic agent, wherein at least a portion of the first sample has a first staining pattern for the first predictive biomarker indicating that at least a portion of the tumor is unlikely to respond to the first therapeutic agent;
(b) a nucleic acid sample obtained by a method comprising:
(b1) excising with an automated dissection tool the portion of the first sample having the staining pattern indicating that the portion of the tumor is unlikely to respond to the first therapeutic agent; and
(b2) extracting the nucleic acid sample from the excised portion of the first sample in a manner compatible with use of the nucleic acid sample in a next generation sequencer; and
(c) one or more additional samples of the tumor, wherein the additional sample(s) are stained for one or more additional predictive biomarker(s) for one or more additional therapeutic agent(s), wherein the additional predictive biomarker(s) correspond(s) to a mutation identified in the nucleic acid sample.

12. The set of diagnostic samples of claim 11, wherein the tumor is a solid tumor.

13. The set of diagnostic samples of claim 12, wherein the solid tumor is a formalin-fixed, paraffin-embedded (FFPE) tissue sample, and the samples of the tumor are microtome sections of the FFPE tissue sample.

14. The set of diagnostic samples of claim 13, wherein sample stained for the additional predictive biomarker(s) is a serial section of the sample stained for the first predictive biomarker.

15. The method of any of claims 1-5, or the system of any of claims 6-10, or the set of diagnostic samples of any of claims 11-14, wherein the next generation sequencer operates on a principle selected from the group consisting of pyrosequencing, cyclic reversible termination, semiconductor sequencing technology, and phospholinked fluorescent nucleotides.

16. The method of any of claims 1-5, or the system of any of claims 6-10, or the set of diagnostic samples of any of claims 11-15, wherein the first predictive biomarker and the additional predictive biomarker(s) are selected from the group consisting of ALK, ATM, BCL2, BRAF, BRCA1, c-KIT, CAIX, CCR4, CD30, Claudin, 17p13.1, DLL3, EGFR1, estrogen receptor, EREG, ERCC1, FGF19, FGFR2b, FGFR3, FOLR1, hyaluronan, HER2/NEU, K-ras, MGMT, MSLN, p53, MDM2, progesterone receptor, PD-L1, PDGFRB, PTEN, and thymidine phosphorylase.

## Patentansprüche

1. Verfahren, umfassend:
histochemisches Färben einer ersten Probe von einem Tumor für einen ersten prognostischen Biomarker für ein erstes Therapeutikum;
Herausschneiden einer oder mehrerer Region(en) aus der ersten Probe mit einem automatisierten Sezierwerkzeug, wobei die herausgeschnittene Region ein Färbungsmuster für den ersten prognostischen Biomarker aufweist, das anzeigt, dass die Region wahrscheinlich nicht auf das erste Therapeutikum ansprechen wird;
Nachweisen einer oder mehrerer Mutation(en), die ein Ansprechen auf ein zusätzliches Therapeutikum oder mehrere zusätzliche Therapeutika prognostiziert/prognostizieren, mit einem Sequenzierungsautomaten der nächsten Generation in einer Nukleinsäureprobe, die aus der/den herausgeschnittenen Region(en) der ersten Probe stammt;
Färben einer zusätzlichen Probe oder mehrerer zusätzlicher Proben von dem Tumor für einen oder mehrere zusätzliche(n) prognostische(n) Biomarker, der/die mit der einen oder den mehreren in den Proben identifizierten Mutation(en) korreliert/korrelieren, wobei der eine oder die mehreren zusätzliche(n) prognostische(n) Biomarker ein Ansprechen auf eines oder mehrere des/der zusätzlichen Therapeutikums/Therapeutika prognostiziert/prognostizieren.

2. Verfahren nach Anspruch 1, ferner umfassend:
Erstellen eines Berichts, der einen therapeutischen Verlauf für das Individuum identifiziert, wobei der therapeutische Verlauf das Verabreichen von Folgendem an das Individuum umfasst:
das erste Therapeutikum, wenn mindestens eine Region der ersten Probe ein Färbungsmuster für den ersten prognostischen Biomarker aufweist, das anzeigt, dass mindestens ein Teil des Tumors wahrscheinlich auf das erste Therapeutikum ansprechen wird; und
ein zusätzliches Therapeutikum oder mehrere zusätzliche Therapeutika, wenn mindestens eine Region der zusätzlichen Probe(n) ein Färbungsmuster für den entsprechenden zusätzlichen prognostischen Biomarker aufweist, das anzeigt, dass mindestens ein Teil des Tumors wahrscheinlich auf das zusätzliche Therapeutikum ansprechen wird.

3. Verfahren nach Anspruch 1 oder 2, wobei der Tumor ein solider Tumor ist.

4. Verfahren nach Anspruch 3, wobei der solide Tumor eine formalinfixierte, paraffineingebettete (FFPE) Gewebeprobe ist und die erste Probe und die zusätzliche(n) Probe(n) Mikrotomschnitte der FFPE-Gewebeprobe sind.

5. Verfahren nach Anspruch 4, wobei die erste Probe und die zusätzliche(n) Probe(n) Serienschnitte sind.

6. System, umfassend:
(a) einen Satz von Objektträgern, umfassend:
(a1) einen ersten Objektträger, auf dem eine erste Probe von einem Tumor aufgebracht ist, wobei die erste Probe histochemisch für einen ersten prognostischen Biomarker für ein erstes Therapeutikum gefärbt ist;
(a2) einen oder mehrere zusätzliche(n) nicht gefärbte(n) Objektträger, auf dem/denen eine zusätzliche Probe von dem Tumor aufgebracht ist;
(b) ein Bildanalysesystem zum Identifizieren einer oder mehrerer Region(en) der ersten Probe mit einem Färbungsmuster für den ersten prognostischen Biomarker, das anzeigt, dass mindestens ein Teil des Tumors wahrscheinlich nicht auf das erste Therapeutikum ansprechen wird;
(c) ein automatisiertes Sezierwerkzeug, das so programmiert ist, dass es die eine oder mehreren Region(en) der ersten Probe mit einem Färbungsmuster für den ersten prognostischen Biomarker, das für ein fehlendes Ansprechen auf das erste Therapeutikum charakteristisch ist, aus der ersten Probe herausschneidet;
(d) einen Sequenzierungsautomaten der nächsten Generation, der so programmiert ist, dass er die Gegenwart oder Abwesenheit von Mutationen, die mit einem oder mehreren zusätzlichen prognostischen Biomarker(n) korrelieren, in einer Nukleinsäureprobe, die aus den Regionen der ersten Probe stammt, die mit dem automatisierten Sezierwerkzeug herausgeschnitten wurden, identifiziert; und
(e) einen automatisierten Objektträgerfärber, der so programmiert ist, dass er den/die zusätzlichen Objektträger mit einem oder mehreren von den zusätzlichen prognostischen Biomarkern färbt.

7. System nach Anspruch 6, ferner umfassend:
(f) ein Laborinformationssystem (LIS), umfassend eine Datenbank, wobei die Datenbank Folgendes enthält:
(f1) eine Mutationsanalyse der Nukleinsäureprobe mit dem Sequenzierungsautomaten der nächsten Generation, wobei die Mutationsanalyse mindestens die Gegenwart oder Abwesenheit von Mutationen in der Nukleinsäureprobe, die mit einem oder mehreren zusätzlichen prognostischen Biomarker(n) für ein zusätzliches Therapeutikum oder mehrere zusätzliche Therapeutika korrelieren, anzeigt; und
(f2) Anweisungen zum Veranlassen des automatisierten Objektträgerfärbers, die zweite Probe des Tumors mit dem einen oder den mehreren zusätzlichen prognostischen Biomarker(n), die durch die Mutationsanalyse identifiziert wurden, zu färben.

8. System nach Anspruch 7, wobei an mindestens einem der nicht gefärbten Objektträger ein Etikett fixiert ist, das von dem LIS erstellt wurde und von dem automatisierten Objektträgerfärber gelesen werden kann, wobei das Etikett den Objektträger als zur Ausführung der Anweisungen von (f2) durch den automatisierten Objektträgerfärber geeignet identifiziert.

9. System nach Anspruch 8, wobei das Etikett den automatisierten Objektträgerfärber automatisch veranlasst, die Anweisungen an der zweiten Probe auszuführen.

10. System nach Anspruch 8, wobei das Etikett einen Bericht für einen Bediener des automatisierten Objektträgerfärbers erstellt, wobei der Bericht den manuellen Bediener anweist, den automatisierten Objektträgerfärber so zu programmieren, dass er die Anweisungen an der zweiten Probe ausführt.

11. Satz von diagnostischen Proben, die aus einem Tumor stammen, wobei der Satz von diagnostischen Proben Folgendes umfasst:
(a) eine erste Probe von einem Tumor, wobei die erste Probe für einen ersten prognostischen Biomarker für ein erstes Therapeutikum gefärbt ist, wobei mindestens ein Teil der ersten Probe ein erstes Färbungsmuster für den ersten prognostischen Biomarker aufweist, das anzeigt, dass mindestens ein Teil des Tumors wahrscheinlich nicht auf das erste Therapeutikum ansprechen wird;
(b) eine Nukleinsäureprobe, die mit einem Verfahren erhalten wird, das Folgendes umfasst:
(b1) Herausschneiden des Teils der ersten Probe mit dem Färbungsmuster, das anzeigt, dass der Teil des Tumors wahrscheinlich nicht auf das erste Therapeutikum ansprechen wird, mit einem automatisierten Sezierwerkzeug; und
(b2) Extrahieren der Nukleinsäureprobe aus dem herausgeschnittenen Teil der ersten Probe in einer mit der Verwendung der Nukleinsäureprobe in einem Sequenzierungsautomaten der nächsten Generation kompatiblen Art und Weise; und
(c) eine oder mehrere zusätzliche Probe(n) von dem Tumor, wobei die zusätzliche(n) Probe(n) für einen oder mehrere zusätzliche(n) prognostische(n) Biomarker für ein zusätzliches Therapeutikum oder mehrere zusätzliche Therapeutika gefärbt ist/sind, wobei der/die zusätzliche(n) prognostische(n) Biomarker einer in der Nukleinsäureprobe identifizierten Mutation entspricht/entsprechen.

12. Satz von diagnostischen Proben nach Anspruch 11, wobei der Tumor ein solider Tumor ist.

13. Satz von diagnostischen Proben nach Anspruch 12, wobei der solide Tumor eine formalinfixierte, paraffineingebettete (FFPE) Gewebeprobe ist und die Proben von dem Tumor Mikrotomschnitte der FFPE-Gewebeprobe sind.

14. Satz von diagnostischen Proben nach Anspruch 13, wobei die für den/die zusätzlichen prognostischen Biomarker gefärbte Probe ein Serienschnitt der für den ersten prognostischen Biomarker gefärbten Probe ist.

15. Verfahren nach einem der Ansprüche 1-5 oder System nach einem der Ansprüche 6-10 oder Satz von diagnostischen Proben nach einem der Ansprüche 11-14, wobei der Sequenzierungsautomat der nächsten Generation nach einem Prinzip arbeitet, das aus der Gruppe ausgewählt ist, die aus Pyrosequenzierung, zyklischer reversibler Termination, Halbleitersequenzierungstechnologie und phosphatgebundenen fluoreszierenden Nukleotiden besteht.

16. Verfahren nach einem der Ansprüche 1-5 oder System nach einem der Ansprüche 6-10 oder Satz von diagnostischen Proben nach einem der Ansprüche 11-15, wobei der erste prognostische Biomarker und der/die zusätzliche(n) prognostische(n) Biomarker aus der Gruppe ausgewählt ist/sind, die aus ALK, ATM, BCL2, BRAF, BRCA1, c-KIT, CAIX, CCR4, CD30, Claudin, 17p13.1, DLL3, EGFR1, Östrogenrezeptor, EREG, ERCC1, FGF19, FGFR2b, FGFR3, FOLR1, Hyaluronan, HER2/NEU, K-ras, MGMT, MSLN, p53, MDM2, Progesteronrezeptor, PD-L1, PDGFRB, PTEN und Thymidinphosphorylase besteht.

## Revendications

1. Procédé comprenant :
la coloration de manière histochimique d'un premier échantillon d'une tumeur pour un premier biomarqueur prédictif pour un premier agent thérapeutique ;
l'excision d'une ou de plusieurs régions du premier échantillon avec un outil de dissection automatisé, dans lequel la région excisée a un motif de coloration pour le premier biomarqueur prédictif indiquant que la région est peu susceptible de répondre au premier agent thérapeutique ;
la détection avec un séquenceur de nouvelle génération d'une ou de plusieurs mutations prédictives d'une réponse à un ou plusieurs agents thérapeutiques supplémentaires dans un échantillon d'acide nucléique dérivé de la ou des régions excisées du premier échantillon ;
la coloration d'un ou de plusieurs échantillons supplémentaires de la tumeur pour un ou
plusieurs biomarqueurs prédictifs supplémentaires corrélés à la ou aux mutations identifiées dans les échantillons, le ou les biomarqueurs prédictifs supplémentaires étant prédictifs d'une réponse à un ou plusieurs des agents thérapeutiques supplémentaires.

2. Procédé selon la revendication 1, comprenant en outre :
la génération d'un rapport identifiant un schéma thérapeutique pour le sujet, ledit schéma thérapeutique comprenant l'administration au sujet :
du premier agent thérapeutique si au moins une région du premier échantillon a un motif de coloration pour le premier biomarqueur prédictif indiquant qu'au moins une partie de la tumeur est susceptible de répondre au premier agent thérapeutique ; et
d'un ou de plusieurs des agents thérapeutiques supplémentaires si au moins une région du ou des échantillons supplémentaires a un motif de coloration pour le biomarqueur prédictif supplémentaire indiquant qu'au moins une partie de la tumeur est susceptible de répondre à l'agent thérapeutique supplémentaire.

3. Procédé selon la revendication 1 ou 2, dans lequel la tumeur est une tumeur solide.

4. Procédé selon la revendication 3, dans lequel la tumeur solide est un échantillon de tissu fixé au formol et inclus en paraffine (FFPE), et le premier échantillon et le ou les échantillons supplémentaires sont des sections au microtome de l'échantillon de tissu FFPE.

5. Procédé selon la revendication 4, dans lequel le premier échantillon et le ou les échantillons supplémentaires sont des sections en série.

6. Système comprenant :
(a) un ensemble de lames de microscope comprenant :
(a1) une première lame de microscope ayant déposé sur celle-ci un premier échantillon d'une tumeur, dans lequel le premier échantillon est coloré de manière histochimique pour un premier biomarqueur prédictif pour un premier agent thérapeutique ;
(a2) une ou plusieurs lames de microscope non colorées supplémentaires ayant déposé sur celles-ci un échantillon supplémentaire de la tumeur ;
(b) un système d'analyse d'image pour l'identification d'une ou de plusieurs régions du premier échantillon ayant un motif de coloration pour le premier biomarqueur prédictif indiquant qu'au moins une partie de la tumeur est peu susceptible de répondre au premier agent thérapeutique ;
(c) un outil de dissection automatisé programmé pour exciser la ou les régions du premier échantillon ayant un motif de coloration pour le premier biomarqueur prédictif caractéristique d'une absence de réponse au premier agent thérapeutique du premier échantillon ;
(d) un séquenceur de nouvelle génération programmé pour identifier la présence ou l'absence de mutations corrélées à un ou plusieurs biomarqueurs prédictifs supplémentaires dans un échantillon d'acide nucléique dérivé des régions du premier échantillon excisées par l'outil de dissection automatisé ; et
(e) un dispositif automatisé de coloration de lame programmé pour colorer la ou les lames supplémentaires avec un ou plusieurs des biomarqueurs prédictifs supplémentaires.

7. Système selon la revendication 6, comprenant en outre :
(f) un système d'information de laboratoire (SIL) comprenant une base de données, la base de données contenant :
(f1) une analyse des mutations de l'échantillon d'acide nucléique par le séquenceur de nouvelle génération, dans lequel l'analyse des mutations indique au moins la présence ou l'absence de mutations dans l'échantillon d'acide nucléique corrélées au ou aux biomarqueurs prédictifs supplémentaires pour le ou les agents thérapeutiques supplémentaires ; et
(f2) des instructions pour diriger le dispositif automatisé de coloration de lame pour colorer le deuxième échantillon de la tumeur avec le ou les biomarqueurs prédictifs supplémentaires identifiés par l'analyse des mutations.

8. Système selon la revendication 7, dans lequel au moins l'une des lames de microscope non colorées a apposé sur celle-ci une étiquette générée par le SIL et lisible par le dispositif automatisé de coloration de lame, dans lequel l'étiquette identifie la lame comme étant appropriée pour l'exécution des instructions de (f2) par le dispositif automatisé de coloration de lame.

9. Système selon la revendication 8, dans lequel l'étiquette dirige automatiquement le dispositif automatisé de coloration de lame pour exécuter les instructions sur le deuxième échantillon.

10. Système selon la revendication 8, dans lequel l'étiquette génère un rapport pour un opérateur du dispositif automatisé de coloration de lame, le rapport donnant l'instruction à l'opérateur manuel de programmer le dispositif automatisé de coloration de lame pour exécuter les instructions sur le deuxième échantillon.

11. Ensemble d'échantillons de diagnostic dérivés d'une tumeur, ledit ensemble d'échantillons de diagnostic comprenant :
(a) un premier échantillon d'une tumeur, dans lequel le premier échantillon est coloré pour un premier biomarqueur prédictif pour un premier agent thérapeutique, dans lequel au moins une partie du premier échantillon a un premier motif de coloration pour le premier biomarqueur prédictif indiquant qu'au moins une partie de la tumeur est peu susceptible de répondre au premier agent thérapeutique ;
(b) un échantillon d'acide nucléique obtenu par un procédé comprenant :
(b1) l'excision avec un outil de dissection automatisé de la partie du premier échantillon ayant le motif de coloration indiquant que la partie de la tumeur est peu susceptible de répondre au premier agent thérapeutique ; et
(b2) l'extraction de l'échantillon d'acide nucléique de la partie excisée du premier échantillon d'une manière compatible avec l'utilisation de l'échantillon d'acide nucléique dans un séquenceur de nouvelle génération ; et
(c) un ou plusieurs échantillons supplémentaires de la tumeur, dans lequel le ou les échantillons supplémentaires sont colorés pour un ou plusieurs biomarqueurs prédictifs supplémentaires d'un ou de plusieurs agents thérapeutiques supplémentaires, dans lequel le ou les biomarqueurs prédictifs supplémentaires correspondent à une mutation identifiée dans l'échantillon d'acide nucléique.

12. Ensemble d'échantillons de diagnostic selon la revendication 11, dans lequel la tumeur est une tumeur solide.

13. Ensemble d'échantillons de diagnostic selon la revendication 12, dans lequel la tumeur solide est un échantillon de tissu fixé au formol et inclus en paraffine (FFPE), et les échantillons de la tumeur sont des sections au microtome de l'échantillon de tissu FFPE.

14. Ensemble d'échantillons de diagnostic selon la revendication 13, dans lequel l'échantillon coloré pour le ou les biomarqueurs prédictifs supplémentaires est une section en série de l'échantillon coloré pour le premier biomarqueur prédictif.

15. Procédé selon l'une quelconque des revendications 1 à 5, ou système selon l'une quelconque des revendications 6 à 10, ou ensemble d'échantillons de diagnostic selon l'une quelconque des revendications 11 à 14, dans lequel le séquenceur de nouvelle génération fonctionne sur un principe choisi dans le groupe constitué par le pyroséquençage, la terminaison cyclique réversible, la technologie de séquençage par semi-conducteurs et les nucléotides fluorescents phospholiés.

16. Procédé selon l'une quelconque des revendications 1 à 5, ou système selon l'une quelconque des revendications 6 à 10, ou ensemble d'échantillons de diagnostic selon l'une quelconque des revendications 11 à 15, dans lesquels le premier biomarqueur prédictif et le ou les biomarqueurs prédictifs supplémentaires sont choisis dans le groupe constitué par ALK, ATM, BCL2, BRAF, BRCA1, c-KIT, CAIX, CCR4, CD30, la claudine, 17p13.1, DLL3, EGFR1, le récepteur des oestrogènes, EREG, ERCC1, FGF19, FGFR2b, FGFR3, FOLR1, l'hyaluronane, HER2/NEU, K-ras, MGMT, MSLN, p53, MDM2, le récepteur de la progestérone, PD-L1, PDGFRB, PTEN et la thymidine phosphorylase.
